# EUROPEAN PATENT SPECIFICATION

(11) **EP 1 744 825 B1**
(45) Date of publication and mention of the grant of the patent: **27.02.2019**
(21) Application number: 05735668.5
(22) Date of filing: 13.04.2005
(51) Int. Cl.: C01B 33/141, C01B 33/20, C01B 33/26, C01B 37/02, C01B 39/02, B01J 13/00, B82Y 30/00, A61K 8/00

(54) **COLLOIDAL COMPOSITIONS AND METHODS OF PREPARING SAME**
KOLLOIDZUSAMMENSETZUNGEN UND HERSTELLUNGSVERFAHREN DAFÜR
COMPOSITIONS COLLOIDALES ET PROCEDES DE PREPARATION DE CELLES-CI

(30) Priority: 19.04.2004 US 827214
(43) Date of publication of application: 24.01.2007
(73) Proprietor: Nalco Company, Naperville, IL 60563-1198 (US)
(72) Inventor: HOLLAND, Brian, T., Oak Park, IL 60304 (US); BATLLO, Francois, Burr Ridge, IL 60527 (US); ORTIZ, Carmen, Y., Woodridge, IL 60517 (US); MACDONALD, Dennis, L., Wheaton, IL 60187 (US)
(74) Representative: Godemeyer Blum Lenze Patentanwälte Partnerschaft mbB - werkpatent
(86) International application number: PCT/US2005/012371
(87) International publication number: WO 2005/104683

(56) References cited:
- WO-A1-94/13584
- US-A- 2 913 419
- US-A- 2 913 419
- US-A- 2 974 108
- US-A- 3 442 795
- US-A- 5 024 826
- US-A- 5 221 497

## Description

### FIELD OF THE INVENTION

The present invention generally relates to production of amorphous colloidal compositions and methods of using the composition. More specifically, the present invention relates to primary amorphous colloidal metalosilicas, characterized by increased pH and hydrothermal stability, which, upon further processing are transformed into mesoporous ZSM-5 nanocrystals.

### BACKGROUND OF THE INVENTION

The preparation and use of colloidal materials, such as colloidal silica, is well-established. Colloidal silica with a metal-coated surface is commercially accepted as a starting material for a variety of uses. Typically, the silica colloid is first synthesized. The colloid is then coated with a metal oxide. During this procedure, both negatively and positively charged surfaces are obtained depending upon the properties of the metallic starting material and the coating method used. Metal containing silica colloids are useful in a multitude of applications, such as chemical mechanical polishing agents in the electronics industry, specialty coating applications, and as support materials in catalytic processes.

Despite its acceptance in the industry, users of silica colloids cannot effectively customize the amount and type of metal component added to the silica particle, because metal addition is limited the surface area and morphology of the silica particle. Moreover, conventional surface-treated silica sols are unstable at neutral pH. i.e., pH 6-8. As exemplified by aluminosilica colloids, aluminum unbound or weakly bound to the colloidal particle surface typically hydrolyze under neutral pH conditions resulting in precipitation or coagulation of the particle coating material. This is particularly problematic for the electronics industry as the demand continues to rise for chemical mechanical polishing slurries that arc stable at neutral pH.

US 2,974,108 relates to aquasols and processes for making them, and is more particularly directed to aquasols comprising negatively charged particles consisting throughout of alumosilica, and is further particularly directed to the step in a process for making such sols, comprising adding an aqueous solution of "active silica" and an aqueous solution of an alkali metal aluminate simultaneously and separately to an aqueous medium containing an amount of alkali sufficient to maintain the pH in the range of from 8 to 12.

US 2,913,419 relates to finely divided products made up of particles which are composed of a skin of silica which contains chemically combined polyvalent metal atoms in a core of another material. It also relates to sols of such particles and to dry particulate solids made of such particles.

Despite increased research for improved metal-containing colloidal compositions, that possess the stability to undergo further processing to, for example, a zeolites catalyst, such commercial parameters remain an unmet need in the industry.

### SUMMARY OF THE INVENTION

High surface area mesoporous zeolites are prepared from metallocolloidal compositions produced from the inventive schemes disclosed herein. Colloidal compositions having metal loadings ranging from as high as about 35 wt% based on silica dispersed within the silica particles are disclosed. The colloidal compositions can further include a stabilizer, such as a quaternary compound, that can facilitate the dispersion and loading of the metal within the silica.

These surface-treated silica colloidal compositions have been synthesized according to the novel method described herein.

A first non-inventive synthetic method of producing a silica colloid provides a first step of producing a stabilizing component in an alkaline solution, followed by adding a silicic acid solution to the alkaline solution, and forming a colloid of silica particles wherein the stabilizing component is dispersed throughout the silicate particle. A cationic metal component is optionally added to the stabilizer-containing alkaline solution. In this scenario, the addition of silicic acid solution to the alkaline solution provides a colloid of silica particles having both the stabilizing component and the metal component homogenously dispersed within one or more of the silicate particles. The resultant silica colloid are amorphous and spherical in shape and carries an increased amounts of metal ranging from about .0001wt% to about 35wt% based on silica. Such compositions are further processed to produce a crystalline structure as described in greater detail below.

In the synthesis procedure according to the invention, a method of preparing a metal-containing silica colloid is provided wherein a silicic acid solution is reacted with a cationic metal component to form a metal silica solution. The metal silica solution is subsequently added to an alkaline solution to form a colloid of metal silica particles. Reacting the silicic acid solution with the metal component forms a metal-silica monomer that is subsequently polymerized as the metal silica solution is added to the alkaline solution. This procedure provides control for location of a metal component within the metal-containing silica colloid. The metal silica solution and the silicic acid solution can be selectively added to the alkaline solution to form a colloid of silica particles containing metal that is dispersed within one or more of the particles. Alternatively, the silicic acid solution can be added to the alkaline solution before the metal silica solution to form a colloid of silica particles having a silica core and metal dispersed within an outer or exterior layer of each particle. Moreover, the metal silica solution and the silicic acid solution can be added to the alkaline solution in an alternating manner to form a colloid of silica particles having a number of layers, wherein the layers alternate between metal containing layers and layers containing only silica in a repeat or successive manner.

An additional synthetic scheme is described herein wherein a colloidal composition is prepared from a heel solution including a stabilizer; preparing a silicic acid solution; and mixing and further processing the heel solution and the silicic acid solution to form the colloidal composition.

A cationic aluminosilica primary particle composition exemplifies one aspect of the invention in which the primary particles are further processed into ZSM-5 nanocrystals. Upon calcination the TPA⁺ is removed and both micropores and mesopores are generated.

### DETAILED DESCRIPTION OF THE PRESENTLY PREFERRED EMBODIMENTS

The present invention generally relates to colloidal compositions and methods of preparing same. As used herein, the term "colloid" and other like terms including "colloidal", "sol", and the like refer to a two-phase system having a dispersed phase and a continuous phase. The colloids of the present invention have a solid phase dispersed or suspended in a continuous or substantially continuous liquid phase, typically an aqueous solution. Thus, the term "colloid" encompasses both phases whereas "colloidal particles" or "particles" refers to the dispersed or solid phase.

More specifically, the present invention relates to colloidal compositions that include silica particles and that can be made in a readily and cost effective manner as described below in greater detail. In general, the present invention provides two types of synthesis procedures. In one synthesis procedure, the present invention utilizes a heel solution that includes a stabilizer, such as a quaternary compound. The stabilizer can enhance the colloidal synthesis in a number of ways, such as by stabilizing and better enabling a metal to be dispersed within the silica particles of the colloidal composition. It is believed that the stabilizer can also enhance the ability of the silica particles to have higher metal loading, such as about 35wt% or less based on silica. In another synthesis procedure, silicic acid and a metal salt solution are selectively added to an alkaline solution thereby producing a colloid that includes silica particles with a metal dispersed therein in a controlled manner. The present invention is now described below in greater detail including specific examples that are illustrative of the compositions and methods of the present invention according to various embodiments without limitation.

In an aspect described herein, a method of preparing a colloidal composition provides adding a silicic acid solution to a reaction vessel that includes a heel solution having an aqueous solution containing a metal component and a stabilizing component to form a colloid of silica particles. In an aspect, the stabilizer is an amine or quaternary compound. Nonlimiting examples of amines suitable for use as the stabilizer include dipropylamine, trimethylamine, triethylmine, tri-n-propylamine, diethanolamine, monoethanolamine, triethanolamine, diisobutylamine, isopropylamine, diisopropylamine, dimethylamine, ethylenediaminetetraacetic acid, pyridine, the like and combinations thereof. Preferably, the stabilizing component is a quaternary amine that forms an alkaline solution when dispersed in water, such as quaternary ammonium hydroxides. In addition, it is further preferred that the quaternary amine includes a tetraalkyl ammonium ion wherein each alkyl group has a carbon chain length of 1 to 10, the alkyl groups being the same or different. Nonlimiting examples of quaternary amines suitable for use as the stabilizer include tetramethylammonium hydroxide (TMAOH), tetrapropylammonium hydroxide (TPAOH), tetraethylammonium hydroxide (TEAOH), tetrabutylammonium hydroxide (TBAOH), tetrahexylammonium hydroxide, tetraoctylammonium hydroxide, tributylmethylammonium hydroxide, triethylmethylammonium hydroxide, trimethylphenylammonium hydroxide, methyltripropylammonium hydroxide, dodecyltrimethylammonium hydroxide, hexadecyltrimethylammonium hydroxide, dimethyldodecylethylammonium hydroxide, diethyldimethylammonium hydroxide, the like and combinations thereof. Also, the bromide and chloride forms of the above mentioned ammonium salts can be used by passing through a hydroxide (anion)-exchange column to produce the alkylammonium hydroxide materials.

The metal can include any suitable material and be derived from any suitable material including metal salts that are soluble or substantially soluble in an aqueous solution. In an embodiment, the metal includes an alkali metal, an alkaline earth metal, a 1^{st} row transition metal, a 2^{nd} row transition metal, a lanthanide, and combinations thereof. Preferred metal components include aluminum, cerium, titanium, tin, zirconium, zinc, copper, nickel, molybdenum, iron, rhenium, vanadium, boron, the like and any combination thereof.

The silicic acid solution can be prepared by passing a sodium silicate solution through a bed of H⁺-cation exchange resin. The resulting deionized silicic acid solution tends to be quite reactive and is typically kept cooled to retard polymerization. Upon addition of the silicic acid solution to the alkaline solution in the heel, the disassociated OH⁻ from the stabilizer catalyzes a polymerization reaction between the cationic metal component and a silica component from the silicic acid to form the colloid of silica particles. The reaction thereby yields a solid phase composed of the metal component, the stabilizer and silica wherein the metal and stabilizer are dispersed within the silica particles. Utilization of the stabilizer component obviates the need to provide a heel containing alkaline catalysts, such as NaOH, KOH, NH₄OH, the like, and combinations thereof. It should be appreciated that any suitable type of silicic acid solution can be utilized.

In addition to catalyzing particle formation, the stabilizer serves as a stabilizing agent for the metal component. Not wishing to be bound to any particular theory, it is believed that the quaternary amine cation interacts with the metal oxide anion in the heel (MO₄^{X-} wherein M is the metal cation) ultimately stabilizing the metal. It is believed that the quaternary amine maintains the metal oxide anion in a four-fold coordination state or tetrahedral orientation so that silicon-to-metal ratios of four can be obtained. Stabilizing the metal component in this manner produces a greater number of silicon-metal linkages allowing the solid phase of the colloid to carry an increased amount of metal compared to surface treated colloids, for example.

In an embodiment, the resultant silica colloid is capable of supporting from about 0.0001wt% to about 35wt% metal based on silica. The metal-stabilized silica solid phase also demonstrates increased stability and remains stable in a pH range of about 1 to about 14. The skilled artisan will appreciate that "stable" means that the solid phase of the colloid is present, dispersed through the medium and stable throughout this entire pH range with effectively no precipitate. The solid phase in an embodiment is amorphous and has a number of particles that are generally spherical in shape. The colloidal particles have a diameter in the range of about 2 nanometers (nm) to about 1000 nm pursuant to an embodiment.

In another procedure described herein, silicic acid is utilized to incorporate or disperse a metal component into the framework of colloidal silica (i.e., doping). The method includes preparing a heel. The heel includes an aqueous solution that at least includes a quaternary amine as defined herein or an alkaline agent. Suitable alkaline agents include, for example, NaOH, KOH, NH₄OH, the like and combination thereof. The silicic acid solution (can be prepared as previously discussed or other suitable manner) is reacted with a cationic metal component to form a metal silicate solution, represented chemically below:

H₄SiO₄ + M^{x+} → [x(H₃SiO₄⁻)-M^{x+}]+xH⁺

The metal silicate solution is subsequently added to the heel to form the colloid. During particle formation, the OH⁻ present in the heel catalyzes the copolymerization of the cationic metal component and silicate (SiO₄⁻) from the silicic acid. This produces a colloid with the metal dispersed within the silica (i.e., incorporated into the particle framework as discussed above), such as having a homogenous distribution of the metal component throughout the entire solid phase of the colloid. Not wishing to be bound by any particular theory, it is believed that the dispersion and loading of the metal is obtained as the copolymerization forms a metal-silica lattice throughout the microstructure of the solid phase. Nonlimiting examples of suitable metals that can be used as the cationic metal component include aluminum, cerium, titanium, tin, zirconium, zinc, copper, nickel, molybdenum, iron, rhenium, vanadium, boron, 1^{st} and 2^{nd} row transition metals, lanthanides, alkali metals, alkaline earth metals, the like and any combination thereof. As previously discussed, the metal component can be derived from any suitable metal source including, for example, any suitable metal salt that is soluble or substantially soluble in an aqueous solution.

According to this synthesis procedure, metal silica colloids of the present invention can have a metal content from about 0.0001% to about 2% by weight based on silica. The metal silica colloids of the present invention are amorphous and generally spherical in shape, wherein the particles have an effective diameter or particle size from about 2 nm to about 1000 nm in an aspect. The metal silica colloids are stable at a pH range from about 1 to about 14, exhibiting effectively no precipitation in this range. The skilled artisan will appreciate that the size of the colloidal particles can be adjusted by varying the addition time of the metal silicate solution to the heel.

As previously discussed, the above-described synthesis procedure can be utilized to effectively control the location of the method and loading thereof within the colloidal particles. In an aspect, the metal silicate solution and the silicic acid solution are selectively added to the heel to control the position of the metal within the solid phase of the colloid as desired. Both silicic acid solution and metal silicate solution can be added to the heel to initiate particle formation or to grow or otherwise increase the size of a pure silica particle initially added to the heel. For example, the metal silicate solution is added to the heel before the silicic acid solution in an aspect. This addition sequence yields a metal containing silica colloid wherein the metal is dispersed in a core or interior layer of the colloidal particle. The subsequent addition of the silicic acid can be used to cover the interior metal-containing portion of the particle with a layer containing on silica without the metal.

Alternatively, the silicic acid solution can be added to the heel prior to the addition of the metal silicate solution in an aspect. This addition sequence yields colloidal particles having a core or interior composed of silica. The metal silicate solution can then be added to coat the silica particle to produce a particle containing metal on an exterior surface or outer layer of the particle wherein the metal is dispersed within this particle layer. The skilled artisan will appreciate the myriad of possibilities available for the composition of the colloid solid phase. Addition of only the metal silicate solution to the heel can yield a colloid having a dispersion or distribution of metal within one or more of the colloidal particle as previously discussed. Adding the metal silicate solution and the silicic acid solution in an alternating manner or a sequence such as metal silicate-silicic acid-metal silicate-silicic acid can yield a colloidal particle having a number of layers wherein metal containing layers are separated by layers containing silica and without a metal in an aspect. It will be appreciated that the duration of silicic acid and/or metal silicate addition can be varied as desired to vary the width or thickness of each particle layer in the colloid. The multiple layered colloid particles of the present invention are generally spherical in shape and have an effective particle size of about 2 nm to about 1000 nm according to an aspect.

It should be appreciated that the colloidal compositions and methods of making same can be modified in any suitable manner. For example, the colloidal compositions as described above can be further processed to form a crystalline structure, such as a crystalline silica, a crystalline metallosilica including a zeolite, the like and combinations thereof. In an embodiment, continued hydrothermal treatment at suitable temperatures and over a suitable period of time can produce a more crystalline silica including metallosilicas, such as zeolites, from the colloidal compositions described-above wherein the colloidal composition includes silica and a stabilizer with or without a metal dispersed within the silica, specific examples of which are provided below in greater detail.

According to an aspect, if the heel in the second synthesis procedure is replaced with an organic cation such as those used in synthesis procedure one (e.g., a stabilizer including tetramethylammonium hydroxide (TMAOH), tetrapropylammonium hydroxide (TPAOH), tetraethylammonium hydroxide (TEAOH) and/or the like), continued hydrothermal treatment after the silicic acid or metal/silicic acid containing solution has been added, can result in the formation of a more crystalline silica or metallosilica including a zeolite.

Doped colloidal silica is useful in multitudinous industrial applications including, for example, dental applications, protein separation, molecular sieves, nanoporous membranes, wave guides, photonic crystals, refractory applications, clarification of wine and juice, chemical mechanical planarization of semiconductor and disk drive components, catalyst supports, retention and drainage aids in papermaking, fillers, surface coatings, ceramic materials, investment casting binders, flattening agents, proppants, cosmetic formulations, particularly sunscreens, and polishing abrasives in the glass, optical and electronics and semiconductor industries. The form of silica used in a particular application depends in large part on the silica particle's size and porosity characteristics. Doped colloidal silica having the desired characteristics is readily prepared according to the method of this invention.

In an aspect, a material for use in an industrial application comprising the colloidal composition is described herein.

In an aspect, the industrial application is selected from the group consisting of catalyst supports, retention and drainage aids in papermaking, fillers, flattening agents, proppants and polishing abrasives.

The present invention will be further understood with reference to the following illustrative examples according to various embodiments without limitation.

### Synthesis Procedure One (not inventive):

A 5 wt% tetramethylammonium hydroxide (20-25 wt%) solution was added to a 12-gallon reactor along with 10.23 wt% of deionized (DI) water. A 0.70 wt% aluminum chlorohydrate (50 wt%) solution was added to 19.82 wt% Dl water. The aluminum chlorohydrate solution was then added to the reactor at room temperature at a rate of 200 mL/min, The reactor was heated to 100 °C. Then, 64.25 wt% silicic acid was added to the reactor at a ramp rate of 100 - 220 mL/min over 3.25 hours. As shown below, Table 1 lists the physical characteristics of the colloidal aluminosilica made in the 12-gallon reactor after it was concentrated by ultra-filtration:

**TABLE 1**

| Concentrated Colloidal Aluminosilica (12 gallon reactor) | Results |
|---|---|
| Solids wt% (specific gravity) | 25.30 |
| Al₂O₃·SiO₂ wt% (ash) | 24.72 |
| Solids wt% (removing water) "includes organic moiety" | 29.75 |
| PH | 11.02 |
| Specific Gravity | 1.1671 |
| Conductance (mhos) | 7100 |
| Particle Size (nm), Titration | 5.00 |
| wt% Al₂O₃ (BOS), ICP | 3.93 |

### Synthesis Procedure Two (according to the invention):

### 1. Preparation of the aluminum containing solutions

### Monomeric containing aluminum solution:

A 0.37 M AlCl₃·6H₂O solution was prepared with a pH of 2.2 and was used as prepared as further described below,

### Polyvalent aluminum containing solution:

A second solution of 0.50 M AlCl₃·6H₂O was prepared. This solution was passed through an ion exchange column containing an anion exchange resin (Dowex 550A (OH⁻)). 100 g of AlCl₃·6H₂O solution was passed through 100 mL of resin. The pH of the aluminum containing solution was ca. 3.4 after being passed through the column. Aluminum chlorohydrate can also be used.

### 2. Preparation of the silicic acid:

25.00 g of (sodium silicate) was added to 57.37 g of DI water. The solution was passed through a column containing a cation exchange resin (Dowex 650C (H⁺)). About 40 mL of resin for 100 g of diluted sodium silicate solution was used to produce a silicic acid solution. To the silicic acid solution, a suitable amount of aluminum containing solution to produce the desired concentration (ppm) of aluminum based on silica (BOS) was added as detailed below in Table 2.

### 3. Preparation of the metallosilica colloids:

Example 1: The silicic acid solution/monomeric aluminum solution (2.93 g of 0.37 M AlCl₃·6H₂O solution) was added to a caustic heel containing 0.30 g of NaOH (50 wt%) in 14.40 g of DI water over a 5.0 hours ramp. A total of 68.57 g of silicic acid solution/aluminum solution was added.
Example 2: The silicic acid solution/polyvalent aluminum solution (3.02 g of 0.50 M AlCl₃·6H₂O anion-exchanged solution) was added to a caustic heel containing 0.30 g of NaOH (50 wt%) in 14.20 g of DI water over a 5.0 hour ramp. A total of 68.57 g of silicic acid solution/aluminum solution was added.
Example 3: The silicic acid solution/polyvalent aluminum solution (3.02 g of 0.50 M AlCl₃·6H₂O anion-exchanged solution) was added to a caustic heel containing 0.30 g of NaOH (50 wt%) in 14.20 g of Example 2 over a 5.0 hour ramp. A total of 68.57 g of silicic acid solution/aluminum solution was added.
Example 4: The silicic acid solution/aluminum solution (3.02 g of 0.50 M AlCl₃·6H₂O anion-exchanged solution) was added to a caustic heel containing 0.30 g of NaOH (50 wt%) in 14.20 g of Example 3 over a 5.0 hour ramp. A total of 68.57 g of silicic acid solution/aluminum solution was added.
Example 5: Pilot Plant synthesis:
   The silicic acid solution/aluminum solution (0.67 g of a 0.87 M solution of aluminum chlorohydrate) was added to a caustic heel containing 0.11 g NaOH (50 wt%) in 3.82 g of 20 nm silica sol in 8.18 g of DI water over a 4.75 hours ramp. The reaction was heated at 93°C. A total of 87.89 g of silicic acid solution/aluminumsolution was added. The final product was cation-exchanged to remove excess sodium, large particle filtered (LPC) and pH adjusted to 6.4.
Example 6: Cerium doped silica colloids:
   A solution of 0.50 M Ce₂(CO₃)₃ was prepared by adding 46g Ce₂(CO₃)₃ into 100ml DI water then adding 1N HCl until dissolved. The solution was then topped up to 200ml with DI water.
   A silicic acid solution was prepared where 200 g of (sodium silicate) was added to 1000 g of DI water. The solution was passed through a column containing a cation exchange resin (Dowex 650C (H⁺)). About 40 mL of resin for 100 g of diluted sodium silicate solution was used.
   To the silicic acid solution, an amount of the cerium containing solution was added to provide the desired concentration (ppm) of cerium based on silica (BOS) as illustrated in Table 2.
   The silicic acid solution/cerium solution (6.2 ml of 0.5 M Ce₂(CO₃)₃ solution) was added to a caustic heel containing 5 g of KOH (45 wt%) in 200 g of DI water over a 5.0 hours ramp. A total of 1200 g of silicic acid solution/cerium solution was added to produce the cerium doped silica colloids
Example 7: Titanium doped silica colloids:
   A titanium containing solution was prepared. In particular, a solution of 0.50 M TiCl₄ was prepared by slowly adding 100ml DI water into a beaker containing 9.4g TiCl₄ and 10ml isopropyl alcohol,
   The silicic acid was prepared in the same fashion as described in Example 6. To the silicic acid was added an amount of the titanium containing solution to produce the desired concentration (ppm) of titanium based on silica (BOS) as illustrated below in Table 2.
   The silicic acid solution/titanium solution (12.6 ml of 0.5 M TiCl₄ solution) was added to a caustic heel containing 5 g of KOH (45 wt%) in 200 g of DI water over a 5.0 hours ramp. A total of 1200 g of silicic acid solution/cerium solution was added to produce the titanium doped silica colloid.
Example 8: Zinc doped silica colloids:
   The zinc containing solution used in this procedure was a commercially-available product, namely 1N Zn(NO₃)₂. The silicic acid was prepared in the same fashion as described in Example 6. To the silicic acid was added an amount of zinc containing solution to provide the desired concentration (ppm) of zinc based on silica (BOS) as illustrated below in Table 2. The silicic acid solution/zinc solution (6 ml of 1 M Zn(NO₃)₂ solution) was added to a caustic heel containing 5 g of KOH (45 wt%) in 200 g of DI water over a 5.0 hours ramp. A total of 1200 g of acid sol/cerium solution was added to produce the zinc doped silica colloid.
   Synthesis Procedure Three (not inventive). Preparation of crystalline silica and metallosilica colloids:
Example 9: Colloidal Silicalite-1 was synthesized with a narrow particle size distribution from a mole composition of:

   1TPAOH:1.9SiO₂:109H₂O

   The source of silica was silicic acid, The reactor vessel was charged with a 20-25 wt% solution of TPAOH, which was heated to 90°C. To this, the silicic acid was added over 3 hours. A clear solution resulted, which was heated for 18 hours.
Example 10: Colloidal ZSM-5 was synthesized with a narrow particle size distribution from a mole composition of:

   65TPAOH:125SiO₂:1Al₂O₃:7000H₂O

The source of silica was silicic acid. The reactor vessel was charged with a 20-25 wt% solution of TPAOH, which was heated to 90°C. To this the aluminum/silicic acid solution was added over 2 hours. A clear solution resulted, which was heated for 24 hours.

### Metallosilica colloids:

Table 2 shows the various prepared metal doped samples with the different heels, pH of the different metal containing solutions, amounts of metal added to the acid sol based on silica (BOS) and a variety of characterization techniques to determine particle size and the extent, if any, agglomeration. As shown below, Table 2 provides a summary of the synthesis procedures according to Examples 1 -10 as detailed above:

**Table 2**

| Sample (Heel) | Metal source and pH | Theoretical and calculated (ICP) amounts of metal BOS^{a} (ppm) | Particle diameter QELS and (TEM) (nm) | Particle diameter (nm) and (surface area) m²/g Titration | Observations | pH final solution [Final wt% metal doped SiO₂] |
|---|---|---|---|---|---|---|
| Example 1 (H₂O/NaOH) | AlCl₃·6H₂O 2.2 | 3,500 (3,281) | 47.8 (29,6) | 12.9 (233) | Prec. | 7.68 [6.54] |
| *Example 2 (H₂O/NaOH) | AlCl₃·6H₂O 3.4 anion-exchanqed | 5,000 (1,508) | 28.5 (273) | 15.3 (196) | No prec. | 9.60 [6.63] |
| *Example 3 | AlCl₃·6H₂O 3.4 anion-exchanged | 5,000 (3,683) | 47.9 (51.1) | 20.9 (143) | No prec. | 9.20 [7.33] |
| *Example 4 | AlCl₃·6H₂O 3,4 anion-exchanged | 5,000 (3,911) | 82.8 (89.9) | 24.3 (123) | No prec. | 9.05 [7.67] |
| Example 5 | Al₂(OH)₅Cl·2H₂O 3,8 | 3,192 (2,446) | 49.3 (53.9) | 29.4 (102) | No prec. | 6.87 [32.7] |
| Example 6 | Ce₂(CO₃)₃ | 11650 | 50.8 | | No prec. | 5.55 |
| Example 7 | TiCl4 | 3985 | 45.7 | | No prec. | 5.42 |
| Example 8 | Zn(NO₃)₂ | 5438 | | | No prec | 5,38 |
| Example 9 | - | - | 241 | | No prec. Silicalite by XRD | 11.5 |
| Example 10 | Al₂(OH)₅Cl·2H₂O 3.8 | 26000 | 292 | | No prec. ZSM-5 byXRD | 11.5 |

| | | | | | | |
|---|---|---|---|---|---|---|
| *Aluminum source is anion exchanged to remove chloride and increase pH. "BOS means based on silica. Example 5 after cation deionization and pH adjusted to 6,87. | | | | | | |

In general, the metal doped colloids described above and made pursuant to various embodiments exhibit good stability in the pH range 3-9. For example, a stability test was conducted on the filtered and cation deionized aluminosilica colloid of Example 5. The pH was adjusted to 4.1, 6.5 and 8.5 and effective particle diameters were measured (QELS) before and after heat treatment for two weeks at 60°C. No gelation occurred with these samples after heat treatment and the particle diameters remained essentially the same as demonstrated below in Table 3:

**TABLE 3**

| pH | Particle Diameter (Initial) (QELS, nm) | Particle Diameter (After Heating @ 60 °C/two weeks) (QELS, nm) |
|---|---|---|
| 4.1 | 496 | 48.6 |
| 6.5 | 49.6 | 49.6 |
| 8.5 | 49.6 | 49.2 |

characterize the meso-ZSM-5 material. SEM was used to determine the structure of the amorphous colloid and meso-ZSM-5. Powder x-ray diffraction (PXRD), TGA, TEM and FT-IR were used to show the presence of ZSM-5, where as nitrogen sorption measurements were used to show the presence of both micropores and mesopores.

### Experimental

### Refluxed Material

Initial primary particles were synthesized from a starting solution of the following molar composition:

20TPAB_{2B}O: 1AlB_{2B}OB_{3B}: 80SiOB_{2B}: 7500HB_{2B}O

The source of silica was silicic acid. The silicic acid was produced by passing a solution containing 25.00 g of sodium silicate in 57.37 g of DI water through a column containing the cation exchange resin, Dowex 650C (H⁺), About 40 mL of resin for 100 g of diluted sodium silicate solution was used. Aluminum chlorohydrate (50 %) solution was used as the source of alumina. The aluminum chlorohydrate was added directly to the silicic acid.

A five-neck reactor vessel equipped with a mechanical stirrer and reflux condenser was charged with a ∼15 wt% solution of TPAOH and heated to 90 °C. To this the silicic acid/aluminum chlorohydrate solution was added over 1.25 h. A clear solution resulted, which was heated under reflux for 30 h. The reaction was monitored over the 30 h by periodically checking particle size with QELS. The material was characterized after 30 h of reflux at 90 °C and after calcining at 550 °C for 5-7 h in air. The reflux material will be designated as 30 h reflux material throughout the text.

### meso-ZSM-5

Further processing of the 30 h reflux material was carried out by transferring a portion to a Teflon-lined autoclave and heating at 100 °C for 1 d and 7 d. The 1 d and 7 d autoclave materials were characterized after heating in the autoclave and after calcining at 550 °C for 5-7 h in air. The autoclave materials will be designated as 1 d and 7 d autoclave materials throughout the text.

### Product Characterization

Particle size analysis of the 30 h refluxed material was carried out with a Coulter N4 Plus Submicron Particle Sizer. Samples were run as is. PXRD on the calcined samples were performed with a Philips PANalytical X'Pert Pro 3040 using Co Kα radiation with a wavelength of 1.78897 Å. Nitrogen sorption measurements were performed with an Autosorb-1C from Quantachrome with micropore capability. Each sample was calcined and then degassed for 16h at 180 °C, except for the samples containing organic which were degassed for 16 h at 120 °C. Each sample was characterized by multi-point BET surface area, total pore volume, t-plot micropore volume and micropore surface area, t-plot external surface area, BJH adsorption pore size distribution and HK micropore size distribution. FT-1R measurements were performed on a Nicolet Model 710 or a Nicolet Avatar 380 instrument by introducing the sample into a KBr pellet. TGA measurements were performed on a TA instrument Model TGA2950 by heating the sample to 1000 °C in air at a rate of 10 °C/min. The SEM micrograph of the 30 h reflux material was taken with a Cambridge 250 Mark III with a Noran Voyager II EDS System. SEM of the 7 d reflux material was taken on a Hitachi FE S4800. The sample was placed on an adhesive conductive carbon disk mounted on an aluminum stud. The sample was coated with 5 nm of Au/Pd. TEM was performed on a JOEI, JEM-2100F Field Emission Electron Microscope. Sample preparation involved sonication in ethanol and dispersing on a holey carbon copper grid. Galbraith Laboratory performed ICP analyses for all samples.

### Results and Discussion

### Reflux Material

A clear solution was obtained after the final addition of silicic acid/aluminum chlorohydrate to the heel containing TPAOH. The reaction was stirred under reflux for an additional 30 h. QELS data shows an almost linear growth from 4 h to 24 h with particle diameters or 15 nm and 232 nm, respectively. A slight decrease in particle diameter is observed after 30 h (216 nm), indicating the end of the reaction in terms of particle growth. Visually, the material begins to show a slight haze at ca. 4 h and then turns increasingly hazy until a white colloidal material remains after 30 h. Similar observations are noticed when colloidal silica particles are grown to large sizes, e.g., > 70 nm in diameter. The 30 h reflux material was also very stable in solution as the beginning of sedimentation took up to 3 months.

A Type IV isotherm was generated which is typical for mesoporous materials. More specifically, the isotherm is similar to those obtained from silica gels generated by a two-step acid-base catalyzed xerogel.{Brinker, 1990 #41} The surface area of the 30 h reflux material was 942 m²/g with a total pore volume of 0.79 cm³/g. These values are also similar to those achieved from two-step acid-base catalyzed xerogels. {Brinker, 1990 #41} In comparison to our TPA⁺ template synthesized 30 h reflux material, a two-step acid-base catalyzed xerogel involves the generation of silica clusters that compact into larger, globular structures upon solvent removal. There are typically two pore sizes associated with xerogel material consisting of micropores from within the silica clusters and mesopores from between the larger, globular structures. Although the 30 h reflux material is generated from the addition of silicic acid/aluminum chlorohydrate to TPA⁺, it may be possible to generate micropores by the removal of TPA⁺ from the small amount of ZSM-5 that is present and mesopores from the soft packing of smaller primary particles that make up the larger colloids. The existence of smaller primary particles may explain the previous QELS data as the larger colloidal particles continue to grow during reflux after all the silicic acid/aluminum chlorohydrate has been added

To explore the possibility of a micropore structure in the calcined 30 h reflux material, t-plot data was generated, along with the HK micropore size distribution plot. The t-plot micropore volume was 0.042 cm³/g, which is significantly lower than the micropore volumes for large particle ZSM-5 materials, ca. 0.15 cm³/g. {Groen, 2004 #44} {Kim, 2003 #7} Similarly, the t-plot micropore surface area of 40 m²/gis considerably lower than ∼300-400 m²/g for large particle ZSM-5 materials. That leaves an extremely high t-plot external surface area of 902 m²/g and pore volume of 0.75 cm³/g for the calcined 30 h reflux material. An HK micropore distribution plot was produced to determine the pore size of the micropores in the 30 h reflux material. The HK plot exhibits a sharp peak at ca. 0.46 nm, which is similar to values obtained by large particle ZSM-5 materials. Unlike large particle ZSM-5 materials, there is BJH adsorption pore size plot of a fairly narrow peak centered at ca. 4.0 nm which is in the mesopore size regime. The higher pore size tailing that is observed on the HK plot for the 30 h reflux material may be due to the overlap of micropores and mesopores.

The data suggests that the majority of the surface area and pore volume is due to mesopores and there appears to be little ZSM-5 generated. To determine if the TPA⁺ plays a role in producing the mesopores, nitrogen sorption data was generated on the noncalcined 30 h reflux material. The noncalcined 30 h reflux material had a BET and t-plot external surface area of 5.40 m²/g with a total pore volume of 0.011 cm³/g, which indicates that almost all of the surface area and pore volume in the calcined material is generated from the removal of TPA⁺. Wang *et al*. showed a similar Type IV isotherm for a self-assembled film of silicalite-1 nanoparticles using TPA⁺ as a template and describe the TPA⁺ as possibly playing a dual role in micropore and mesopore templating.

The colloidal compositions of the present invention can be utilized in a number of different and suitable types of applications in any suitable forms and amounts thereof. For example, the colloidal composition can be used as a chemical mechanical polishing agent including use for electronic components; a catalyst material and supports thereof including use in the petrochemical industry, such as cracking to increase fractions of gasoline; as a detergent or agent thereof to remove calcium ions and/or the like from solution: and any other types of suitable applications.

## Claims

1. A method of forming a colloidal silica composition, the method comprising the steps of:
preparing a silicic acid solution, a metal salt solution and an alkaline solution;
mixing and further processing the silicic acid solution and the metal salt solution with the alkaline solution;
selectively adding the metal salt solution and the silicic acid solution to the alkaline solution to form a colloid of silica particles containing the metal;
forming one or more silica particles doped with a metal wherein the metal is dispersed within one or more of the silica particles, and wherein the solid phase of the metal silica colloid is substantially amorphous;
wherein the step of selectively adding the metal salt solution and the silicic acid solution to the alkaline solution to form a colloid of silica particles containing the metal comprises one of the following alternative steps:
a) adding the metal salt solution before the silicic acid solution and forming the colloid of silica particles wherein the metal is dispersed within an interior layer of one or more of the silica particles, or
b) adding the silicic acid solution before the metal salt solution and forming the colloid of silica particles wherein the metal is dispersed within an outer layer of one or more of the silica particles, or
c) adding the metal salt solution and the silicic acid solution in an alternating manner and forming the colloid of silica particles having a metal-containing layer and a non-metal containing layer.

2. The method of claim 1, wherein the silica particles doped with metal include about 2 wt % of the metal based on silica.

3. The method of claim 1, wherein the metal is selected from the group consisting of an alkali metal, an alkaline earth metal, a first row transition metal, a second row transition metal, a lanthanide, and combinations thereof.

4. Use of a method according to any of claims 1 to 3 for controlling a location of a metal within a metal-containing silica colloid

5. The use of claim 4, wherein in alternative c) one or more of the silica particles includes a layered structure that has the non-metal containing layer disposed on the metal containing layer in a repeat manner.

## Patentansprüche

1. Verfahren zum Bilden einer kolloidalen Siliziumdioxidzusammensetzung, wobei das Verfahren folgende Schritte umfasst:
Herstellen einer Kieselsäurelösung, einer Metallsalzlösung und einer Alkalilösung;
Mischen und Weiterverarbeiten der Kieselsäurelösung und der Metallsalzlösung mit der Alkalilösung;
selektives Zugeben der Metallsalzlösung und der Kieselsäurelösung zu der Alkalilösung unter Bildung eines Kolloids aus Siliziumdioxidteilchen, die das Metall enthalten;
Bilden eines oder mehrerer Siliziumdioxidteilchen(s), die mit einem Metall dotiert sind, wobei das Metall in einem oder mehreren der Siliziumdioxidteilchen dispergiert ist, und
wobei die Festphase des Metall-Siliziumdioxidkolloids im Wesentlichen amorph ist;
wobei der Schritt des selektiven Zugebens der Metallsalzlösung und der Kieselsäurelösung zu der Alkalilösung, unter Bildung eines Kolloids aus Siliziumdioxidteilchen, die das Metall enthalten, einen der folgenden alternativen Schritte umfasst.
a) Zugeben der Metallsalzlösung vor der Kieselsäurelösung und Bilden des Kolloids von Siliziumdioxidteilchen, wobei das Metall in einer inneren Schicht aus einem oder mehreren der Siliziumdioxidteilchen dispergiert ist, oder
b) Zugeben der Kieselsäurelösung vor der Metallsalzlösung und Bilden des Kolloids von Siliziumdioxidteilchen, wobei das Metall in einer äußeren Schicht aus einem oder mehreren der Siliziumdioxidteilchen dispergiert ist, oder
c) Zugeben der Metallsalzlösung und der Kieselsäurelösung in einer abwechselnden Weise und Bilden des Kolloids von Siliziumdioxidteilchen mit einer metallhaltigen Schicht und einer nichtmetallhaltigen Schicht.

2. Verfahren nach Anspruch 1, wobei die Siliziumdioxidteilchen, die mit Metall dotiert sind, etwa 2 Gew.-% des Metalls basierend auf Siliziumdioxid beinhalten.

3. Verfahren nach Anspruch 1, wobei das Metall aus der Gruppe bestehend aus einem Alkalimetall, einem Erdalkalimetall, einem Übergangsmetall der ersten Reihe, einem Übergangsmetall der zweiten Reihe, einem Lanthanid und Kombinationen davon ausgewählt ist.

4. Verwendung eines Verfahrens nach einem der Ansprüche 1 bis 3 zum Steuern der Position eines Metalls innerhalb eines metallhaltigen Siliziumdioxidkolloids.

5. Verwendung nach Anspruch 4, wobei alternativ c) eines oder mehrere der Siliziumdioxidteilchen eine Schichtstruktur beinhalten, bei der die nichtmetallhaltige Schicht in einer abwechselnden Weise auf der metallhaltigen Schicht angeordnet ist.

## Revendications

1. Procédé de formation d'une composition de silice colloïdale, le procédé comprenant les étapes de :
préparation d'une solution d'acide silicique, d'une solution de sel métallique et d'une solution alcaline ;
mélange et traitement ultérieur de la solution d'acide silicique et de la solution de sel métallique avec la solution alcaline ;
ajout de manière sélective de la solution de sel métallique et de la solution d'acide silicique à la solution alcaline pour former un colloïde de particules de silice contenant le métal ;
formation d'une ou plusieurs particules de silice dopées par un métal, le métal étant dispersé dans une ou plusieurs des particules de silice, et la phase solide du colloïde de silice métallique étant sensiblement amorphe ;
l'étape d'ajout de manière sélective de la solution de sel métallique et de la solution d'acide silicique à la solution alcaline pour former un colloïde de particules de silice contenant le métal comprenant l'une des étapes alternatives suivantes :
a) l'ajout de la solution de sel métallique avant la solution d'acide silicique et la formation du colloïde de particules de silice, le métal étant dispersé dans une couche intérieure d'une ou plusieurs des particules de silice, ou
b) l'ajout de la solution d'acide silicique avant la solution de sel métallique et la formation du colloïde de particules de silice, le métal étant dispersé dans une couche extérieure d'une ou plusieurs des particules de silice, ou
c) l'ajout de la solution de sel métallique et de la solution d'acide silicique d'une manière alternative et la formation du colloïde de particules de silice possédant une couche contenant du métal et une couche ne contenant pas de métal.

2. Procédé selon la revendication 1, dans lequel les particules de silice dopées par un métal comprennent environ 2 % en poids du métal à base de silice.

3. Procédé selon la revendication 1, dans lequel le métal est sélectionné dans le groupe composé d'un métal alcalin, d'un métal alcalino-terreux, d'un métal de transition de premier rang, d'un métal de transition de second rang, d'un lanthanide et de combinaisons de ceux-ci.

4. Utilisation d'un procédé selon l'une quelconque des revendications 1 à 3 pour commander un emplacement d'un métal dans un colloïde de silice contenant du métal.

5. Utilisation selon la revendication 4, dans lequel dans l'alternative c), une ou plusieurs des particules de silice comprennent une structure en couches dont la couche ne contenant pas de métal est disposée de manière répétée sur la couche contenant du métal.
